# EUROPÄISCHE PATENTSCHRIFT

(11) **EP 4 012 406 B1**
(45) Veröffentlichungstag und Bekanntmachung des Hinweises auf die Patenterteilung: **22.05.2024**
(21) Anmeldenummer: 20213901.0
(22) Anmeldetag: 14.12.2020
(51) Int. Cl.: G01N 33/46

(54) **SYSTEM UND EIN VERFAHREN ZUR BESTIMMUNG DER FEUCHTE IN EINER HOLZSTRUKTUR**
SYSTEM AND METHOD FOR DETERMINING HUMIDITY IN A WOOD STRUCTURE
SYSTÈME ET PROCÉDÉ DE DÉTERMINATION DE L'HUMIDITÉ DANS UNE STRUCTURE EN BOIS

(43) Veröffentlichungstag der Anmeldung: 15.06.2022
(73) Patentinhaber: Elascon GmbH, 79183 Waldkirch (DE)
(72) Erfinder: RUPPRECHT, Holger, 79183 Waldkirch (DE)
(74) Vertreter: Fischer, Michael

(56) Entgegenhaltungen:
- EP-A1- 0 709 814
- BR-A- PI0 603 495
- DE-A1-102011 108 764

## Beschreibung

Die vorliegende Erfindung bezieht sich auf ein System und ein Verfahren zur Bestimmung der Feuchte in einer Holzstruktur, insbesondere einer statisch tragenden Holzstruktur, wie z.B. eines Gebäudedachs, einer Brücke und dergleichen.

In der Konstruktion von neuen Häusern, Werkhallen und in der Altbausanierung besteht ein hoher Bedarf an statisch einwandfreien Verbindungen von Holzbalken/Holzrahmen mit Betonelementen und/oder vorgefertigten Holz/Betonverbundelementen. Derartige Beton- und/oder Holz/Betonverbundelemente werden mit den Holzbalken/Holzrahmen zu sogenannten Holz/Beton-Verbundtragwerken zusammengefügt und werden dann beispielsweise als Wände, tragende Decken und/oder (Fertig-)Dachelemente eingesetzt. Diese Holz/Beton-Verbundtragwerke haben gegenüber dem reinen Holzbau den Vorteil, dass sie trotz des Betonanteils dennoch vergleichsweise leicht sind und trotz des Holzanteils dennoch noch eine genügend hohe Wärmespeichermasse aufweisen. Weiter verbessern derartige Tragwerke die akustischen Eigenschaften eines Gebäudes entscheidend und haben ebenfalls eine gute Feuerwiderstandsdauer. Mit dem verbesserten Schallschutzverhalten verringern sie zudem spürbare Schwingungen von Böden und steifen somit das Gebäude statisch hervorragend aus. Abgesehen von diesen technischen Vorteilen befriedigen die sichtbaren Holzbalken auch die ästhetischen Wünsche der Bauherrschaft.

Ein besonderes Augenmerk ist auf die Festigkeit von Holzstrukturen allgemein, wie z.B. als Trägstruktur für Brücken, Dächer und dergleichen, wie auch von Holz/Betonverbundelementen mit zumindest teilweise tragenden Holzstrukturen, wie z.B. Bodenbalken, Deckenbalken, Ständerbalken und dergleichen, zu legen. Als hygroskopischer Baustoff steht Holz in einer ständigen Verbindung zu der das Holz umgebenden Atmosphäre. So nimmt Holz Feuchtigkeit aus der Umgebungsluft auf, wenn die Luftfeuchtigkeit steigt. Sinkt die Luftfeuchtigkeit wieder, gibt das Holz entsprechend Feuchtigkeit ab. Liegt jedoch die Holzfeuchtigkeit dauerhaft über etwa 20 %, besteht die Gefahr eines Befalls von holzzersetzenden Pilzen, die die Holzsubstanz dauerhaft schädigen und eine verringerte Holzfestigkeit hervorrufen können. Bei Holzfeuchtigkeiten unterhalb von 5 % kann es vermehrt zu Rissbildung und Delamination von Leimfugen in Holzstrukturen kommen. Daher sollten statisch relevante Bauteile aus Holz langfristig in einem Feuchtigkeitsbereich zwischen 5 - 20 % liegen, um die Standsicherheit zu gewährleisten. Über die Beobachtung der Holzfeuchte können deshalb wichtige Rückschlüsse auf den Zustand der Holzsubstanz besonders in statisch tragenden Strukturen geschlossen werden.

Das Monitoring von (Holz-)Bauwerken ist nach jetzigem Stand wenig entwickelt. Derzeit ist es bekannt, die folgenden Messmethoden einzusetzen:
a) Messung des elektrischen Widerstandes;
b) Verformungsmessung mit Dehnungsmessstreifen;
c) Verformungsmessung mit faser-optischen Sensoren;
d) Ultraschallmessung, und
e) Röntgen-Untersuchung.

Außer der Methode der Messung des elektrischen Widerstandes werden diese Messmethoden heute noch intensiv auf Machbarkeit und Aussagekraft untersucht und sind nicht im Stadium der Marktreife. Weiter ist aus der deutschen Patentanmeldung DE 10 2011 108764 A1 eine Vorrichtung zur Messung der Feuchte von Holz bekannt, bei zumindest eine Aussenwand des Gehäuses der Vorrichtung zu Anlage an dem Holz ausgebildet ist, sodass sich das Innere des Gehäuses zumindest teilweise mit dem Holz in einem Feuchteaustausch befindet. Leider bildet diese Vorrichtung, auch wenn sie in die Holzstruktur eingeschraubt ist, nicht die exakt herrschenden thermodynamischen Verhältnisse im Innern der Holzstruktur ab, was ebenso auch für die Lehre der EP 0 709 814 A1 gilt, wo eine Compound-Struktur verwendet wird, um den in ein Loch des Konstruktionsmaterials eingesetzten Leitfähigkeitssensor zu verschliessen.

Der vorliegenden Erfindung liegt daher die Aufgabe zugrunde, ein Verfahren und ein System zur Bestimmung der Holzfeuchte in einer Holzstruktur anzugeben, die sich durch einen einfachen Aufbau der erforderlichen Komponenten und ihre einfache Umsetzbarkeit bei gleichzeitig gewünscht guter Aussagekraft aufgrund der Einstellung eines echte thermodynamischen Gleichgewichts für einen Feuchtesensor im Innern der Holzstruktur auszeichnet.

Diese Aufgabe wird bezüglich des Verfahrens erfindungsgemäss durch ein Verfahren zur Bestimmung der Holzfeuchte in einer Holzstruktur gelöst, umfassend die folgenden Schritte:
a) Bohren eines Loches in die Holzstruktur;
b) Einsetzen eines Feuchtesensors in das Loch, wobei der Feuchtesensor einen zumindest teilweise mit Löchern versehenen Hüllkörper und einen darin angeordneten Hohlraum aufweist;
c) Verschliessen des Loches mit einem Holzzapfen, der vorzugsweise eine Dicke von 10 bis 25 mm aufweist; und
d) Messen der Luftfeuchtigkeit in dem Hohlraum, der sich weitgehend in einem thermodynamischen Gleichgewicht mit dem das Loch umgebenden Holz der Holzstruktur befindet;
e) drahtloses Übertragen des gemessenen Wertes für die Luftfeuchtigkeit an ein Daten-Gateway; und
f) Auswerten des gemessenen Wertes für die Luftfeuchtigkeit an einer von dem Feuchtesensor räumlich abgesetzten Auswerteeinheit.

Auf diese Weise misst der eigentliche Sensor die Luftfeuchtigkeit in einem gasdicht von der Umgebung abgedichteten Hohlraum, der sich mit dem umgebenden Holz im Wesentlichen in einem thermodynamischen Gleichgewicht befindet. Zugleich sorgt aber der für den Verschluss des Lochs gewählte Holzzapfen dafür, das auch über den Holzzapfen selbst der Feuchteaustausch mit der Umgebung gewährleistet ist, sodass so die im Innern der Holzstruktur herrschenden thermodynamischen Verhältnisse durch den in das Loch eingesetzten Sensor und den das Loch verschliessenden Holzzapfen nicht gestört werden. Die drahtlose Übertragung der Messwerte aus dem Hohlraum gewährleistet eine Auswertung der Messdaten zur Bestimmung der Holzfeuchte aus der Entfernung.

Die Auswertung des für die Luftfeuchtigkeit in dem Hohlraum gemessenen Wertes kann weiter verbessert werden, wenn zusätzlich zur Luftfeuchtigkeit auch die Temperatur in dem Hohlraum gemessen und übertragen wird.

Ein guter Kompromiss zur Erzielung einer hinreichenden Einbautiefe für den Feuchtesensor ergibt sich, wenn der Holzzapfen eine Dicke von 10 bis 25 mm, vorzugsweise im Bereich von 15 mm, aufweist. Damit ergibt sich eine ebenso dicke Überdeckung des Feuchtesensors mit dem Holz der zu überwachenden Holzstruktur.

In einer vorteilhaften Weiterbildung der Erfindung kann der Holzzapfen das Loch gasdicht verschliessen und dabei vorzugsweise bündig mit der Holzstruktur abschliessend in das Loch eingesetzt werden. Der eingesetzte Holzzapfen unterscheidet sich dann rein äusserlich nicht von einem Holzzapfen, der z.B. zum Ersatz eines Astlochs oder dergleichen in die Holzstruktur eingesetzt, dabei in der Regel eingeklebt, wird.

Zur Aufrechthaltung des Gasdichtheit des durch den Holzzapfen abgeschlossenen Loches ist es vorteilhaft, wenn mit dem Einsetzen des Holzzapfen die Faserrichtung des Holzzapfen mit einem Winkel zur Faserrichtung des das Loch umgebenden Holz gesetzt wird, der vorzugsweise im Bereich von 90° liegt.

Weiter kann der Feuchtesensor von einer in den Feuchtesensor integrierten Batterie elektrisch versorgt werden und/oder durch eine induktive Kopplung berühungslos aufgeladen bzw. versorgt werden.

Zur Einstellung besondere realer Umgebungsbedingungen für den in das Loch eingesetzten Feuchtesensor ist es vorteilhaft, wenn das Holz des Holzzapfen der Holzart des das Loch umgebenden Holzes entsprechend gewählt wird.

Bezüglich des Systems wird die o.g. Aufgabe erfindungsgemäss durch die Merkmale des Patentanspruchs 7 in zum Verfahren analoger Weise gelöst. Weitere vorteilhafte Ausgestaltungen der vorliegenden Erfindung können den übrigen Unteransprüche 8 bis 12 entnommen werden.

Bevorzugte Ausführungsbeispiele der vorliegenden Erfindung werden anhand einer Zeichnung näher erläutert. Dabei zeigen:
- Figur 1: schematisch die Sorptionskurve verschiedener Bauhölzer bei 20°C;
- Figur 2: schematisch einen Aufbau eines Holzfeuchtesensors mit Datenübertragungskette;
- Figur 3: schematisch eine Einbaulage des Holzfeuchtesensors gemäss Figur 2 in einer tragenden Holzstruktur;
- Figur 4: gemittelter Holz- und Luftfeuchteverlauf über einen Messzeitraum aufgeschlüsselt nach Holzart und Sensorart; und
- Figur 5: Kalibrierungsfunktion von Sensoren aufgeschlüsselt nach Holzart und Sensorart.

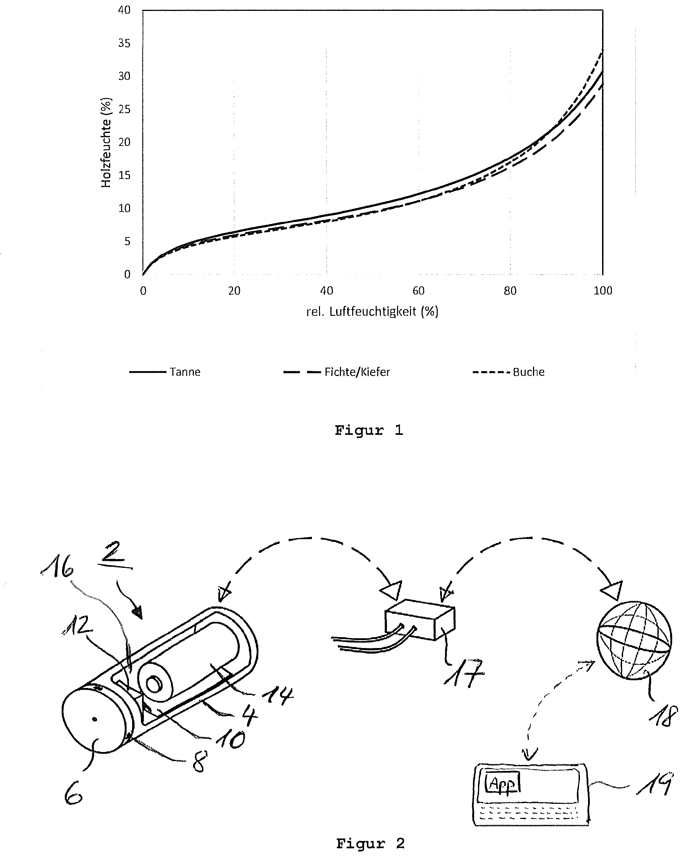

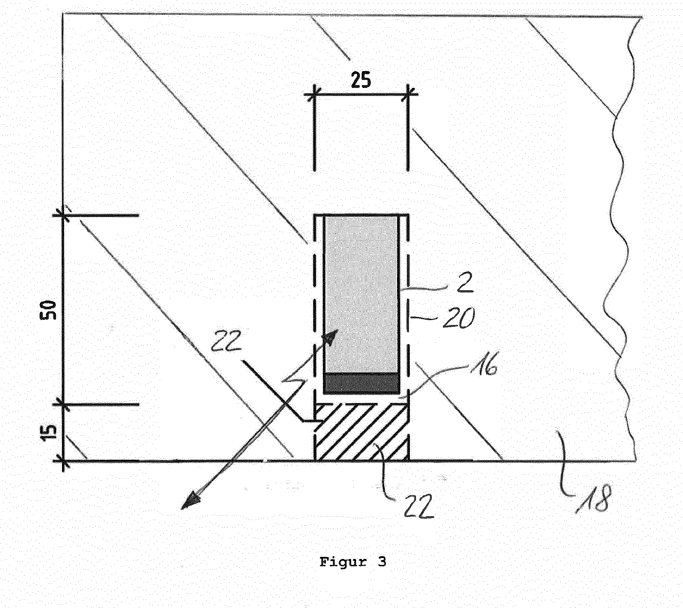

Figur 2 zeigt in schematischer Ansicht einen Feuchtesensor 2, der eine äussere zylindrische Kunststoffhülle 4 aufweist, hier beispielsweise vorliegend mit einem Durchmesser von 21,0 mm und einer Höhe von 47,0 mm. Ein stirnseitiger Verschluss 6 dieser Kunststoffhülle 4 weist vier ovale Öffnungen 8 mit etwa 1,0 mm Durchmesser auf, durch die ein Gasaustausch mit dem umgebenden Holz 18 erfolgt, wenn der Feuchtesensor 2 in einem Hohlraum (Topfloch 20 in Figur 3) im Holz 18 eingesetzt ist (siehe Figur 3). Hinter diesem Verschluss 6 befindet sich eine Sensorplatine 10 mit einem Sensor, der die Luftfeuchtigkeit und die Temperatur in diesem im Innern des Gehäuses befindlichen Hohlraum 16 in regelmäßigen Abständen misst. Zur Verbreitung dieser Messdaten weist der Feuchtesensor 2 ein Funkmodul 12 auf, das wie der Sensor 10 von einer in den Feuchtesensor 2 integrierten Batterie 14 gespeist wird. Per Funk werden diese Messdaten dann an ein zentrales Gateway 17, wie z.B. ein in einem Gebäude angeordneten WLAN-Router oder ein an einer tragenden Holzstruktur verankertes Mobilfunkmodul, gesendet. Dieses Gateway 17 macht die gemessenen Informationen daher online im Internet 18 zugänglich, sodass beispielsweise von einer auf einem Smart Phone oder eine PDA-Gerät 19 installierten Applikation App auf die Daten zwecks entsprechender Auswertung zugegriffen werden kann.

Im vorliegenden Ausführungsbeispiel wird der Feuchtesensor 2 durch die Batterie 14 gespeist, sodass der Einbau kabellos erfolgen kann. Um die Entladung der Batterie 14 verfolgen zu können, können die Messdaten auch noch Informationen über den Ladezustand der Batterie 14 umfassen. Alternativ oder ergänzend zur Batterie 14 könnte der Feuchtesensor 2 auch entsprechende Ladespulen aufweissen, sodass er von aussen her durch ein entsprechendes Treibersignal berühungslos aufgeladen werden kann. Besonders bei einem Einsatz draussen, wo der Feuchtesensor 2 sehr kalten Witterungsbedingungen ausgesetzt sein kann, könnte dieses Konzept der berühungslosen Aufladung Vorteile gegenüber einer Batterie bringen, zumal an solchen im Freien befindlichen Infrastrukturen, wie z.B. Holzbrücke für Fussgänger und Radfahrer, draussen sehr häufig Strom aus dem öffentlichen Netz zur Beleuchtung des Fahrweges oder auch über Solarpanel erzeugter Strom verfügbar gemacht werden kann.

Wie in Figur 3 gezeigt, wird der Feuchtesensor 2 in einen mit einem Holzzapfen 22 abgeschlossenen Hohlraum (Loch 20) im Holz 18, zum Beispiel einer statisch tragenden Holzstruktur, eingebracht und von außen mit dem Holzzapfen 22 verschlossen. Das Mikroklima in diesem abgeschlossenen Hohlraum und damit im Hohlraum 16 innerhalb des Gehäuses 4 des Feuchtesensors 2 wird ausschließlich durch das umliegende Holz 18 konditioniert. Dadurch kann bei einer gemessenen erhöhten Luftfeuchtigkeit in diesem Hohlraum 16 auf eine gestiegene Holzfeuchtigkeit geschlossen werden und so beispielsweise eine Gefährdung für die Bausubstanz in Form des Holzes 18 frühzeitig erkannt werden. Gründe für eine stark geändert Holzfeuchte können beispielhaft sein:
a) direkt anstehendes Wasser in flüssiger Form (bspw. bei Defekt der Abdichtungslage);
b) große Temperatur- und Luftfeuchteschwankungen (bspw. bei ungedämmten Dächern); und
c) Nutzungsänderung eines Gebäudes.

Wie in Figur 3 gezeigt, kann beim Einbau des Feuchtesensors 2 wie folgt vorgegangen werden. In dem zu überwachenden Bereich des Holzes 18 wird beispielsweise mit einem Forstnerbohrer (Ø 25 mm) ein etwa 65 mm tiefes Topfloch 20 quer zur Faser des Holzes 18 gebohrt. Das Topfloch 20 wird ausgeblasen und der Feuchtesensor 2 wird darin eingesetzt. Anschließend wird das Topfloch 20 mit einem Holzzapfen 22 bündig verschlossen. Gemäss aktuellen Versuchsauswertungen scheint es für die Genauigkeit der Messungen dabei keine Rolle zu spielen, ob der Feuchtesensor 2 zuerst mit dem stirnseitigen Verschluss 6 oder zuerst mit der dem stirnseitigen Verschluss 6 gegenüberliegenden Rückseite in das Topfloch 20 eingeführt wird. Um die Montage zu vereinfachen, kann der Feuchtesensor 2 auch an der im eingebauten Zustand dann innenliegenden Seite des Holzzapfens 22 befestigt werden, beispielsweise durch Verklebung mit einem Cyanacrylat-Klebstoff. Vorzugsweise besteht der Holzzapfen aus der gleichen Holzart wie das zu prüfende Holz 18. Der Holzzapfen 22 hat hier vorliegend eine Länge von etwa 15 mm und einen Durchmesser von etwa 25 mm. Während der Desorption schwindet der Holzzapfen 22 geringfügig.

Damit die Fuge zwischen Holzzapfen 22 und Lochleibung möglichst dampfdicht bleibt, wird die Faserrichtung des Holzzapfens 22 um einen Winkel verdreht zur Faserrichtung des zu überwachenden Holzes 18 eingebaut. Dabei kann dieser Winkel beispielsweise auch 90° betragen, sodass die Faserrichtung des Holzzapfen 22 und die Faserrichtung des zu prüfenden Holzes 18 im Bereich des Topfloches 20 annähernd senkrecht zueinanderstehend verlaufen. Auf diese mindestens leicht winkelverdrehte Einbauweise des Holzzapfens 22 kann es vermieden werden, dass Längsholz an Längsholz anliegt, da dieses ein deutlich höheres Schwindmass aufweist als die im Winkel dazu verlaufende Faserrichtung. Ergänzend oder alternativ kann jede Art von geeignetem Zapfen auch noch einen aussen umlaufenden Dichtring und/oder aussen umlaufende die Eindruckrichtung begünstigende Profile aufweisen, sodass die Fuge zwischen Zapfen und Lochleibung auch sicher dampfdicht verschlossen werden kann.

Für die Geschwindigkeit der Detektion eines möglichen Defekts an dem Holz 18 - auch Holzstruktur 18 genannt - ist die Einbautiefe des Feuchtesensors 2 ein wichtiges Kriterium. Bei zu geringer Holzdeckung reagiert der Feuchtesensor 2 bereits auf kurzzeitige Änderungen der Holzfeuchte an der Oberfläche der Holzstruktur 18. Ist der Feuchtesensor 2 aber zu tief verbaut, macht sich die isolierende Wirkung des Holzes 18 bemerkbar und eine Schädigung der Holzsubstanz wird erst verzögert erkannt. Eine Holzdeckung von etwa 10 bis 25 mm, vorzugsweise im Bereich von 15 mm, die durch die Länge des Holzzapfens 22 sichergestellt wird, scheint nach den bisherigen Erkenntnissen eine relativ gute Holzdeckung (Einbautiefe) zu sein. Im Hohlraum (Topfloch 20) ist der Feuchtesensor 2 seitlich von einer 2,0 mm dicken Luftschicht umgeben, stirnseitig am Verschluss beträgt die Luftschichtstärke etwa 3,0 mm.

Neben der neu entwickelten, energie-effizienten Hardware für den Feuchtesensor 2 ist das Knowhow, anhand der erhaltenen Informationen eine zuverlässige Aussage über den Schädigungsfortschritt am Holz 18 (Bauwerk) zu treffen, von zentraler Bedeutung. Mit dem Wissen, wie die Feuchesensoren 2 in welchen Holzbauteilen verbaut sind, können die gemessenen Werte besonders interpretiert werden und Rückschlüsse auf das Ausmaß und Fortschreiten eines Schadens gezogen werden.

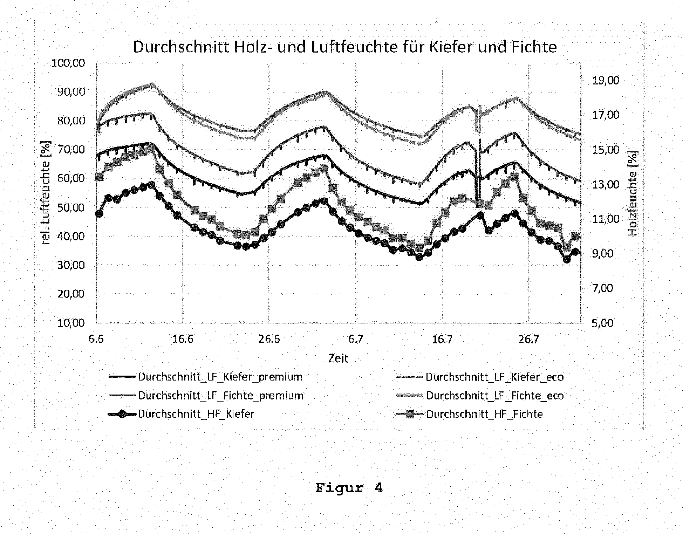

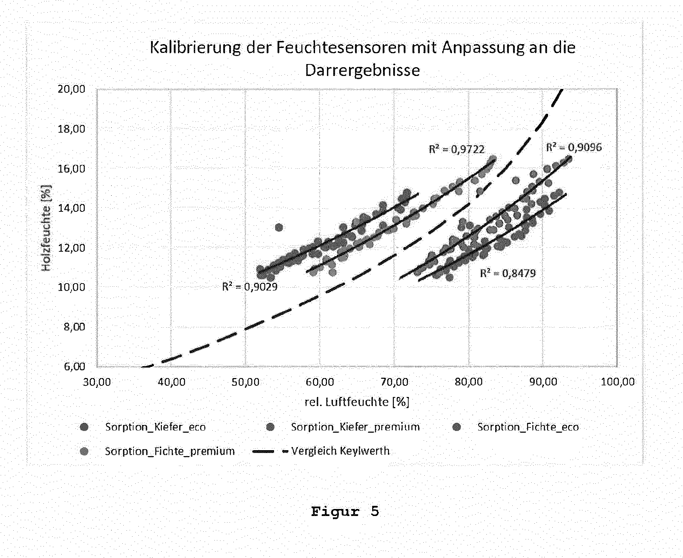

Nachfolgend befinden sich Auswertungen aus Versuchsreihen zu dem vorstehend beschriebenen Feuchtesensor 2 (vgl. Figuren 4 und 5). In Figur 4 sind die gemittelten Messergebnisse über den Messzeitraum dargestellt. Bei einer konstanten Temperatur von 50°C wurde in dieser Zeit die Luftfeuchtigkeit zwischen 50 % und 90 % variiert. Die oberen vier Graphen stellen die Mittelwerte für zwei unterschiedliche Sensortypen "eco" und "premium" in Abhängigkeit der Holzart dar. Die unteren beiden Graphen zeigen die vergleichend gemessene Holzfeuchte für Kiefern- und Fichtenholz. Es zeigt sich eine deutliche Korrelation zwischen gemessener Luftfeuchte der Feuchtesensoren 2 und der effektiven Holzfeuchte. Die Holzfeuchte wurde dabei nach dem eingangs erwähnten Verfahrens des elektrischen Widerstandes gemessen.

In Figur 5 sind diese Ergebnisse als Sorptionskurve dargestellt. Mit dieser Sorptionskurve kann zukünftig aus der gemessenen Luftfeuchte und Temperatur auf die Holzfeuchte geschlossen werden. Vergleichend ist gestrichelt eine Sorptionskurve aus der Literatur wie in Abbildung 1 dargestellt. Die Gradienten der Graphen aus den Versuchen ähneln dem der Literatur. Dadurch besteht die Möglichkeit bereits vorhandene Sorptionskurven auf die Feuchtesensoren 2 umzurechnen.

Das hier vorgestellte Verfahren zur Positionierung der Feuchtesensoren 2 decken den gleichen Messbereich ab, wie dies mit der Messung der Holzfeuchte auf Basis des elektrischen Widerstandes möglich ist. Es kann gezielt die Holzfeuchte an neuralgischen Punkten des Bauwerks überwacht werden. Die genaue Festlegung der Messpunkte basiert auf Erfahrung aus Schadensbildern und Versuchen und ist daher von fachkundigem Personal durchzuführen.

Der Einbau der Feuchtesensoren 2 ist jedoch deutlich einfacher als der Messaufbau bei der Messung des elektrischen Widerstandes. Mit vorgefertigtem Werkzeug kann dies durch ortsansässige holzverarbeitende Betriebe wie Zimmerer oder Schreiner erfolgen. Darüber hinaus ist das Auslesen der Daten deutlich vereinfacht, da keine Kabelanbindung notwendig ist. Auf die Daten besteht ein Fernzugriff (IoT) und die Auswertung kann als Service vom Hersteller selbst übernommen werden. Zusätzlich ist der Verschluss mit einem Holzzapfen 22 optisch ansprechend und kann so auch in sichtbaren Bereichen verwendet werden und ermöglicht so die Aufrechterhaltung des in dem Bohrloch 20 herrschenden Mikroklimas, weil der Holzzapfen dieselben Eigenschaften bezüglich eines Feuchteaustausches mit der Umgebung aufweist, wie die das Bohrloch 20 umgebende Holzstruktur 18 selbst auch.

## Patentansprüche

1. Verfahren zur Bestimmung der Holzfeuchte in einer Holzstruktur (18), umfassend die folgenden Schritte:
a) Bohren eines Loches (20) in die Holzstruktur (18);
b) Einsetzen eines Feuchtesensors (2) in das Loch (20), wobei der Feuchtesensor (2) einen zumindest teilweise mit Löchern (8) versehenen Hüllkörper (4) und einen darin angeordneten Hohlraum (16) aufweist;
c) Verschliessen des Loches (20) mit einem Holzzapfen (22), der vorzugsweise eine Dicke von 10 bis 25 mm, aufweist; und
d) Messen der Luftfeuchtigkeit in dem Hohlraum (16), der sich weitgehend in einem thermodynamischen Gleichgewicht mit dem das Loch (20) umgebenden Holz der Holzstruktur (18) befindet;
e) drahtloses Übertragen des gemessenen Wertes für die Luftfeuchtigkeit an ein Daten-Gateway (17); und
f) Auswerten des gemessenen Wertes für die Luftfeuchtigkeit an einer von dem Feuchtesensor räumlich abgesetzten Auswerteeinheit (19) .

2. Verfahren nach Anspruch 1,
**dadurch gekennzeichnet, dass**
zusätzlich zur Luftfeuchtigkeit auch die Temperatur in dem Hohlraum (16) gemessen und übertragen wird.

3. Verfahren nach Anspruch 1 oder 2,
**dadurch gekennzeichnet, dass**
der Holzzapfen (22) das Loch (20) gasdicht verschliesst und vorzugsweise bündig mit der Holzstruktur (18) abschliessend eingesetzt wird.

4. Verfahren nach einem der Ansprüche 1 bis 3,
**dadurch gekennzeichnet, dass**
mit dem Einsetzen des Holzzapfen (22) die Faserrichtung des Holzzapfen (22) mit einem Winkel zur Faserrichtung der das Loch umgebenden Holzstruktur (18) gesetzt wird, der vorzugsweise im Bereich von 90° liegt.

5. Verfahren nach einem der Ansprüche 1 bis 4,
**dadurch gekennzeichnet, dass**
der Feuchtesensor (2) von einer in den Feuchtesensor (2) integrierten Batterie (14) elektrisch versorgt wird und/oder durch eine induktive Kopplung berühungslos aufgeladen bzw. versorgt wird.

6. Verfahren nach einem der Ansprüche 1 bis 5,
**dadurch gekennzeichnet, dass**
das Holz des Holzzapfen (22) der Holzart der das Loch (20) umgebenden Holzstruktur (18) entsprechend gewählt wird.

7. System zur Bestimmung der Holzfeuchte in einer Holzstruktur (18), umfassend:
a) die Holzstruktur (18) und ein in die Holzstruktur (18) gebohrtes Loch (20);
b) einen in das Loch (20) eingesetzten Feuchtesensor (2), wobei der Feuchtesensor (2) einen zumindest teilweise mit Löchern (8) versehenen Hüllkörper (4) und einen darin angeordneten Hohlraum (16) aufweist;
c) einen das Loch (20) verschliessenden Holzzapfen (22), der vorzugsweise eine Dicke von 10 bis 25 mm, aufweist; und
d) einen Sensor (10) zur Messung der Luftfeuchtigkeit in dem Hohlraum (16), der sich weitgehend in einem thermodynamischen Gleichgewicht mit dem das Loch (20) umgebenden Holz der Holzstruktur (18) befindet;
e) im Feuchtesensor (2) angeordnete Mittel (12) zum drahtlosen Übertragen des gemessenen Wertes für die Luftfeuchtigkeit an ein Daten-Gateway (17); und
f) eine räumlich von dem Feuchtesensor (2) abgesetzte Auswerteeinheit (19) zur Bestimmung der Holzfeuchte aus den empfangenen Werten für die von dem Feuchtesensor (2) gemessene Luftfeuchtigkeit.

8. System nach Anspruch 7,
**dadurch gekennzeichnet, dass**
zusätzlich zur Luftfeuchtigkeit auch die Temperatur in dem Hohlraum (16) messbar und übertragbar ist.

9. System nach Anspruch 7 oder 8,
**dadurch gekennzeichnet, dass**
der Holzzapfen (22) das Loch (20) gasdicht verschliesst und vorzugsweise bündig mit der Holzstruktur (18) abschliessend eingesetzt ist.

10. System nach einem der Ansprüche 7 bis 9,
**dadurch gekennzeichnet, dass**
mit dem Einsetzen des Holzzapfen (22) die Faserrichtung des Holzzapfen (22) mit einem Winkel zur Faserrichtung der das Loch (20) umgebenden Holzstruktur (18) gesetzt wird, der vorzugsweise im Bereich von 90° liegt.

11. System nach einem der Ansprüche 7 bis 10,
**dadurch gekennzeichnet, dass**
der Feuchtesensor (2) von einer in den Feuchtesensor (2) integrierten Batterie (14) elektrisch versorgbar ist und/oder durch eine induktive Kopplung berühungslos aufladbar bzw. versorgbar ist.

12. System nach einem der Ansprüche 7 bis 11,
**dadurch gekennzeichnet, dass**
das Holz des Holzzapfen (22) der Holzart der das Loch (20) umgebenden Holzstruktur (18) entsprechend gewählt ist.

## Claims

1. Method for determining the wood moisture in a wood structure (18), comprising the following steps:
a) drilling a hole (20) into the wood structure (18);
b) inserting a moisture sensor (2) into the hole (20), wherein the moisture sensor (2) has an enclosure body (4), which is at least partially provided with holes (8), and a cavity (16) arranged therein;
c) closing off the hole (20) using a wooden peg (22), which preferably has a thickness of 10 to 25 mm; and
d) measuring the air humidity in the cavity (16), which is largely in a thermodynamic equilibrium with the wood, surrounding the hole (20), of the wood structure (18);
e) wirelessly transferring the measured value for the air humidity to a data gateway (17); and
f) evaluating the measured value for the air humidity on an evaluation unit (19) that is spatially offset from the moisture sensor.

2. Method according to claim 1,
**characterised in that**
in addition to the air humidity, the temperature in the cavity (16) is also measured and transferred.

3. Method according to claim 1 or 2,
**characterised in that**
the wooden peg (22) closes off the hole (20) in a manner impermeable to gas and is preferably inserted flush with the wood structure (18).

4. Method according to one of claims 1 to 3,
**characterised in that**
when the wooden peg (22) is inserted, the fibre direction of the wooden peg (22) is set with an angle preferably lying in the region of 90° in relation to the fibre direction of the wood structure (18) that surrounds the hole.

5. Method according to one of claims 1 to 4,
**characterised in that**
the moisture sensor (2) is supplied with electricity from a battery (14) integrated into the moisture sensor (2) and/or is charged or supplied in a contactless manner by way of inductive coupling.

6. Method according to one of claims 1 to 5,
**characterised in that**
the wood of the wooden peg (22) is chosen to match the type of wood of the wood structure (18) surrounding the hole (20).

7. System for determining the wood moisture in a wood structure (18), comprising:
a) the wood structure (18) and a hole (20) drilled into the wood structure (18);
b) a moisture sensor (2) inserted into the hole (20), wherein the moisture sensor (2) has an enclosure body (4), which is at least partially provided with holes (8), and a cavity (16) arranged therein;
c) a wooden peg (22), which closes off the hole (20) and preferably has a thickness of 10 to 25 mm; and
d) a sensor (10) for measuring the air humidity in the cavity (16), which is largely in a thermodynamic equilibrium with the wood, surrounding the hole (20), of the wood structure (18);
e) means (12) arranged in the moisture sensor (2) for wirelessly transferring the measured value for the air humidity to a data gateway (17); and
f) an evaluation unit (19) that is spatially offset from the moisture sensor (2) for determining the wood moisture from the received values for the air humidity measured by the moisture sensor (2).

8. System according to claim 7,
**characterised in that**
in addition to the air humidity, the temperature in the cavity (16) can also be measured and transferred.

9. System according to claim 7 or 8,
**characterised in that**
the wooden peg (22) closes off the hole (20) in a manner impermeable to gas and is preferably inserted flush with the wood structure (18).

10. System according to one of claims 7 to 9,
**characterised in that**
when the wooden peg (22) is inserted, the fibre direction of the wooden peg (22) is set with an angle preferably lying in the region of 90° in relation to the fibre direction of the wood structure (18) that surrounds the hole (20).

11. System according to one of claims 7 to 10,
**characterised in that**
the moisture sensor (2) can be supplied with electricity from a battery (14) integrated into the moisture sensor (2) and/or can be charged or supplied in a contactless manner by way of inductive coupling.

12. System according to one of claims 7 to 11,
**characterised in that**
the wood of the wooden peg (22) is chosen to match the type of wood of the wood structure (18) surrounding the hole (20).

## Revendications

1. Procédé de détermination de l'humidité du bois dans une structure (18) en bois, comprenant les stades suivants :
a) perçage d'un trou (20) dans la structure (18) en bois ;
b) insertion d'un capteur (2) d'humidité dans le trou (20), le capteur (2) d'humidité ayant un corps (4) d'enveloppe pourvu au moins en partie de trous (8) et une cavité (16), qui y est disposée ;
c) fermeture du trou (20) par un tampon (22) en bois, qui a, de préférence une épaisseur de 10 à 25 mm ; et
d) mesure de l'humidité de l'air dans la cavité (16), qui se trouve, dans une grande mesure, en équilibre thermodynamique avec le bois, entourant le trou (20), de la structure (18) en bois ;
e) transmission sans fil de la valeur mesurée de l'humidité de l'air à une passerelle (17) de données ; et
f) évaluation de la valeur mesurée de l'humidité de l'air sur une unité (19) d'évaluation, à distance dans l'espace du capteur d'humidité.

2. Procédé suivant la revendication 1,
**caractérisé en ce que**
supplémentairement à l'humidité de l'air, on mesure et on transmet également la température dans la cavité (16).

3. Procédé suivant la revendication 1 ou 2,
**caractérisé en ce que**
le tampon (22) en bois ferme le trou (20) d'une manière étanche au gaz et est inséré avec fermeture, de préférence au ras de la structure (18) en bois.

4. Procédé suivant l'une des revendications 1 à 3,
**caractérisé en ce que**
par l'insertion du tampon (22) en bois, on met la direction des fibres du tampon (22) en bois sous un angle avec la direction des fibres de la structure (18) en bois, entourant le trou, qui est, de préférence de l'ordre de 90°.

5. Procédé suivant l'une des revendications 1 à 4,
**caractérisé en ce que**
l'on alimente électriquement le capteur (2) d'humidité par une batterie (14) intégrée dans le capteur (2) d'humidité et/ou on le charge ou l'on alimente sans contact par un couplage inductif.

6. Procédé suivant l'une des revendications 1 à 5,
**caractérisé en ce que**
l'on choisit le bois du tampon (22) en bois d'une manière correspondante au type de bois de la structure (18) en bois, entourant le trou (20).

7. Système de détermination de l'humidité du bois dans une structure (18) en bois, comprenant :
a) la structure (18) en bois et un trou (20) percé dans la structure (18) en bois ;
b) un capteur (2) d'humidité inséré dans le trou (20), le capteur (2) d'humidité ayant un corps (4) d'enveloppe pourvu au moins en partie de trous (8) et une cavité (16), qui y est disposée ;
c) un tampon (22) en bois fermant le trou (20), qui a, de préférence une épaisseur de 10 à 25 mm ; et
d) un capteur (10) de mesure de l'humidité de l'air dans la cavité (16), qui se trouve, dans une grande mesure, en équilibre thermodynamique avec le bois, entourant le trou (20), de la structure (18) en bois ;
e) un moyen (12) disposé dans le capteur (2) d'humidité pour la transmission sans fil de la valeur mesurée de l'humidité de l'air à une passerelle (17) de données ; et
f) une unité (19) d'évaluation à distance dans l'espace du capteur (2) d'humidité pour la détermination de l'humidité du bois à partir des valeurs reçues de l'humidité de l'air mesurées par le capteur (2) d'humidité.

8. Système suivant la revendication 7,
**caractérisé en ce que**
supplémentairement à l'humidité de l'air, également la température dans la cavité (16) peut être mesurée et transmise.

9. Système suivant la revendication 7 ou 8,
**caractérisé en ce que**
le tampon (22) en bois ferme le trou (20) de manière étanche au gaz et est inséré avec fermeture, de préférence au ras de la structure (18) en bois.

10. Système suivant l'une des revendications 7 à 9,
**caractérisé en ce que**
par l'insertion du tampon (22) en bois, on met la direction des fibres du tampon (22) en bois sous un angle avec la direction des fibres de la structure (18) en bois, entourant le trou (20), qui est, de préférence de l'ordre de 90°.

11. Système suivant l'une des revendications 7 à 10,
**caractérisé en ce que**
le capteur (2) d'humidité peut être alimenté électriquement par une batterie (14) intégrée dans le capteur (2) d'humidité et/ou peut être chargée ou alimentée sans contact par un couplage inductif.

12. Système suivant l'une des revendications 7 à 11,
**caractérisé en ce que**
le bois du tampon (22) en bois est choisi de manière à correspondre au type de bois de la structure (18) en bois, entourant le trou (20) .
